# EUROPEAN PATENT SPECIFICATION

(11) **EP 2 633 081 B1**
(45) Date of publication and mention of the grant of the patent: **11.01.2017**
(21) Application number: 11836728.3
(22) Date of filing: 27.10.2011
(51) Int. Cl.: C12Q 1/68, G01N 33/50

(54) **PROXIMITY LIGATION TECHNOLOGY FOR WESTERN BLOT APPLICATIONS**
NÄHERUNGSLIGATIONSTECHNOLOGIE FÜR WESTERN-BLOT-ANWENDUNGEN
TECHNOLOGIE DE LIGATURE DE PROXIMITÉ POUR DES APPLICATIONS DE WESTERN BLOT

(30) Priority: 10.11.2010 US 411955 P; 29.10.2010 US 407944 P
(43) Date of publication of application: 04.09.2013
(73) Proprietor: Olink Bioscience AB, 751 24 Uppsala (SE)
(72) Inventor: LANDEGREN, Ulf, S-756 46 Uppsala (SE); HAGNER-MCWHIRTER, Åsa, S-751 84 Uppsala (SE); IVANSSON, Daniel, S-751 84 Uppsala (SE)
(74) Representative: Dzieglewska, Hanna Eva
(86) International application number: PCT/SE2011/051279
(87) International publication number: WO 2012/057689

(56) References cited:
- WO-A1-01/61037
- WO-A1-2012/049316
- WO-A2-2009/037659
- SODERBERG O ET AL: "Characterizing proteins and their interactions in cells and tissues using the in situ proximity ligation assay", METHODS : A COMPANION TO METHODS IN ENZYMOLOGY, ACADEMIC PRESS INC., NEW YORK, NY, US, vol. 45, no. 3, 1 July 2008 (2008-07-01), pages 227-232, XP023437838, ISSN: 1046-2023, DOI: 10.1016/J.YMETH.2008.06.014 [retrieved on 2008-07-11]
- FREDRIKSSON S ET AL: "Protein detection using proximity-dependent DNA ligation assays", NATURE BIOTECHNOLOGY, NATURE PUBLISHING GROUP, NEW YORK, NY, US, vol. 20, 1 May 2002 (2002-05-01), pages 473-477, XP002375040, ISSN: 1087-0156, DOI: 10.1038/NBT0502-473
- J. GORANSSON ET AL: "A single molecule array for digital targeted molecular analyses", NUCLEIC ACIDS RESEARCH, vol. 37, no. 1, 1 January 2009 (2009-01-01), pages e7-e7, XP055069029, ISSN: 0305-1048, DOI: 10.1093/nar/gkn921
- IRENE WEIBRECHT ET AL: "Proximity ligation assays: a recent addition to the proteomics toolbox", EXPERT REVIEW OF PROTEOMICS, FUTURE DRUGS LTD., LONDON, GB, vol. 7, no. 3, 1 January 2010 (2010-01-01) , pages 401-409, XP008163362, ISSN: 1478-9450, DOI: 10.1586/EPR.10.10
- GUSTAFSDOTTIR S M ET AL: "Proximity ligation assays for sensitive and specific protein analyses", ANALYTICAL BIOCHEMISTRY, ACADEMIC PRESS INC, NEW YORK, vol. 345, no. 1, 1 October 2005 (2005-10-01), pages 2-9, XP027219350, ISSN: 0003-2697 [retrieved on 2005-09-21]
- SÖDERBERG O ET AL: "Direct observation of individual endogenous protein complexes in situ by proximity ligation", NATURE METHODS, NATURE PUBLISHING GROUP, GB, vol. 3, no. 12, 1 December 2006 (2006-12-01), pages 995-1000, XP002522344, ISSN: 1548-7091, DOI: 10.1038/NMETH947 [retrieved on 2006-10-29]
- ZHOU HEPING ET AL: "Two-color, rolling-circle amplification on antibody microarrays for sensitive, multiplexed serum-protein measurements", GENOME BIOLOGY, BIOMED CENTRAL LTD., LONDON, GB, vol. 5, no. 4, 30 March 2004 (2004-03-30), page R28, XP021012886, ISSN: 1465-6906, DOI: 10.1186/GB-2004-5-4-R28
- SAKANYAN V ET AL.: 'High-throughput and multiplexed protein array technology: protein-DNA and protein-protein interactions' JOURNAL OF CHROMATOGRAPHY B vol. 815, 2005, pages 77 - 95, XP027627433
- JARVIUS M ET AL.: 'In situ Detection of Phosphorylated Platelet-derived Growth Factor Receptor beta Using a Generalized Proximity Ligation Method' MOLECULAR & CELLULAR PROTEOMICS vol. 6, 2007, pages 1500 - 1509, XP002635677
- SODERBERG O. ET AL.: 'Direct observation of individual endegenous protein complexes in situ by proximity ligation' NATURE METHODS vol. 3, 2006, pages 995 - 1000, XP002522344
- GORANSSON J ET AL.: 'A single molecule array for digital targeted molecular analyses' NUCLEIC ACIDS RESEARCH vol. 37, no. E7, 2009, pages 1 - 9, XP055069029
- GULLBERG M ET AL.: 'A sense of closeness: protein detection by proximity ligation' CURRENT OPINION IN BIOTECHNOLOGY vol. 14, 2003, pages 82 - 86, XP026832023
- WEIBRECHT IRENE ET AL.: 'Proximity ligation assays: a recent addition to the proteomics toolbox' EXPERT REVIEW OF PROTEOMICS vol. 7, 2010, pages 401 - 409, XP008163362
- LIU Y ET AL.: 'Western Blotting via Proximity Ligation for High Performance Protein Analysis, O111.011031' MOLECULAR & CELLULAR PROTEOMICS vol. 10, 2011, pages 1 - 9, XP009155198

## Description

### Field of the Invention

The present invention relates to methods for Western blot type of analysis. More specifically, the invention relates to a Western blot method which incorporates the advantages of the proximity ligation technology. Also provided is a multiplexed Western blot method using the proximity ligation technology.

### Background of the Invention

### Western Blotting

Western blotting (or, protein immunoblotting) is an analytical technique used to detect specific proteins in a given sample of tissue homogenate, cell lysate or other protein containing samples. It uses gel electrophoresis to separate native or denatured proteins by the length of the polypeptide (denaturing conditions) or by the 3-D structure of the protein (native/ non-denaturing conditions). The proteins are then transferred to a membrane (typically nitrocellulose or PVDF), where they are probed (detected) using antibodies specific to the target protein (see http://en.wikipedia.org/wiki/Western_blot). Another option similar to Western blotting is to perform a Dot Blot or reverse phase protein microarray assay (RPMA), were the protein samples are directly spotted onto a membrane without an electrophoresis separation step. For certain applications, the proteins are probed in the gel without a transfer step. However, further description of Western blot methodology is based on protein samples transferred to a membrane. During the detection process the membrane is probed for the protein of interest by the use of an antibody specific for the protein of interest. Due to possibilities of increased signal amplification and to avoid negative effects on target specific affinity related to primary antibody conjugation, this traditionally takes place in a two-step process (using a primary target specific antibody and a secondary labeled antibody specific for the primary antibody), although there are now one-step detection methods available for certain applications. The one-step method allows the process to occur faster and with a lower amount of consumables, but sensitivity may be compromised. This requires a probe antibody which both recognizes the protein of interest and contains a detectable label, probes which are often available for known protein tags. The primary probe is incubated with the membrane in a manner similar to that for the primary antibody in a two-step process, and is then ready for direct detection after a series of wash steps.

There are a number of detection methods available for Western blotting, here illustrated for secondary detection formats.

The colorimetric detection method depends on incubation of the Western blot with a substrate that reacts with the reporter enzyme (such as peroxidase or alkaline phosphatase) that is conjugated to the secondary antibody. This converts the soluble dye into an insoluble form of a different color that precipitates next to the enzyme and thereby stains the membrane. Development of the blot is then stopped by washing away the soluble dye. Protein levels are evaluated through densitometry (how intense the stain is) or spectrophotometry.

Chemiluminescent detection methods depend on incubation of the membrane/blot with a substrate that will luminesce when exposed to the reporter enzyme on the secondary antibody. The light signal is then detected by photographic film, and more recently by CCD cameras which capture a digital image of the membrane/blot. Image analysis software is used to quantify the signals and for calculation of molecular weights (MW) if a MW standard was run on the gel together with the samples.

Radioactive labeled secondary antibodies are also used and the signals are detected by using X-ray film of phospho imaging screens. The importance of radioactive detections methods is declining, because it is very expensive, health and safety risks are high and ECL provides a useful alternative (see http://en.wikipedia.org/wiki/Western_blot).

Fluorescently labeled secondary antibodies are detected by fluorophore specific excitation wavelength and emission filter settings using a laser or CCD based imager. Fluorescence is considered to be among the most sensitive detection methods for blotting analysis and has the advantage of multiplexing possibilities, enabling detection of targets of the same molecular weight (overlaid signals distinguished by using separate detection channels).

### Proximity Ligation Assay

*In-situ* PLA (proximity ligation assay) technology was developed by Ulf Landegren *et al. (In situ* detection of phosphorylated PDGF receptor β using a generalized proximity ligation method; Jarvius, M., et al.; Mol Cell Proteomics. 2007 Sep; 6(9):1500-9. Epub 2007 Jun 12) and commercialized by Olink Biosciences AB (www.olink.se). *In-situ* PLA offers extreme signal amplification and the possibility to count individual binding events (when microscope read-out is used). Via the optional use of dual recognition events at the primary level, the specificity is highly increased. This detection principle has been applied to interrogation of fixed tissue/cells (immunihistochemistry-like applications) and to a lesser extent protein arrays.

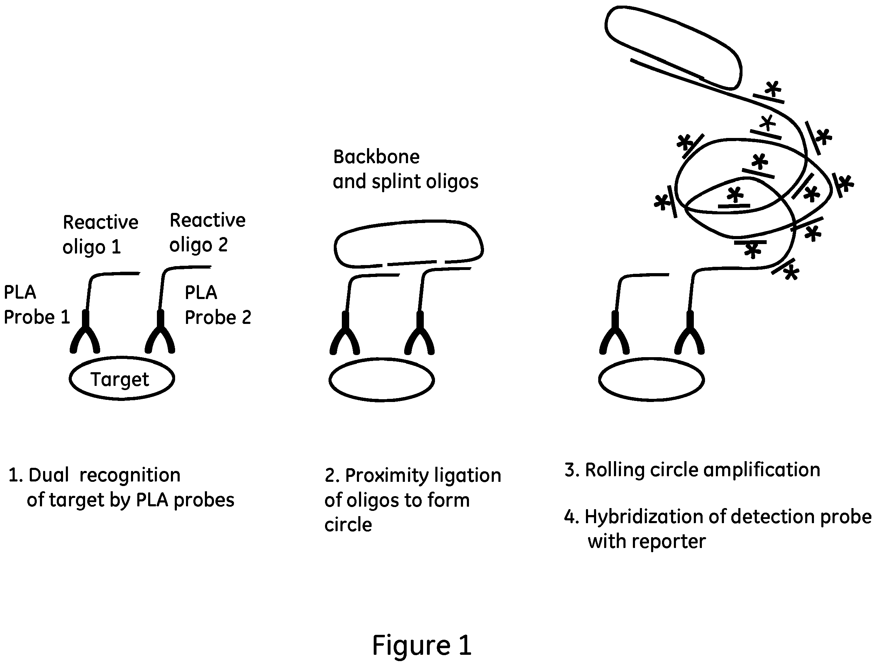

In the standard design, two affinity-binders (antibodies, affibodies, aptamers etc.) are conjugated to sequence-designed oligonucleotides and used to probe a sample (Figure 1). If and only if the two reagents bind in proximity of each other a paired set of specialized and sequence matched oligonucleotides (i.e. backbone- and splint oligo) can hybridize to the binder-conjugated oligos and be converted to a circular molecule by ligation reactions. Next, rolling circle amplification (RCA) is used to elongate one of the binder-conjugated oligos. As a result, each correctly bound pair of affinity reagents are converted into localized DNA-spheres (∼1µm in diameter, also referred to as rolling circle products or RCPs) containing up to a thousand copies of the circular DNA molecule (engineered to contain binding sites for oligonucleotide reporter probes). The detection is accomplished through hybridization of detection oligos. More advanced designs requiring three or more oligo-conjugated affinity-reagents binding in proximity for circularization/RCA have also been reported (Protein Diagnostics by Proximity Ligation: Combining Multiple Recognition and DNA Amplification for Improved Protein Analyses, Leuchowius, K-L., et al., Molecular Diagnostics (Second Edition), 2010, Pages 299-306).

There are several possible implementations of the standard design, for example: (1) A single primary antibody in combination with two oligo-conjugated secondary antibodies. The secondary antibodies specifically recognize two distinct epitopes of the primary antibody (species specific and/or conjugated haptens such as biotin). (2) Two primary antibodies in combination with two oligo-conjugated secondary antibodies. Primary antibodies need to be of different species origin or conjugated to different haptens. (3) Two oligo-conjugated primary antibodies.

### Summary of the Invention

In a first aspect of the invention, it is provided a method for detecting a protein in a sample, wherein said protein has been separated in an electrophoretic gel or on a blotting membrane, said method comprising:
a) providing a said gel or a said membrane containing a said protein from said sample;
b) providing a proximity probe pair, each probe comprising a binding moiety with affinity for a different binding site on the said protein and a reactive oligonucleotide, coupled thereto;
c) binding said proximity probes to their respective binding sites on said protein through the binding moiety;
d) adding a splint oligonucleotide and a backbone oligonucleotide which are complementary to the reactive oligonucleotide pair, and allow them to hybridize to the reactive oligonucleotides, such that when said probes in said proximity probe pair have both bound to said protein the respective ends of the splint oligonucleotide are hybridised directly in contact with the ends of the backbone oligonucleotide;
e) ligating, using a DNA ligase, the hybridized backbone and splint oligonucleotides to create a circularized DNA molecule wherein said circularized DNA molecule is formed from the backbone and splint oligonucleotides only when said probes in said proximity probe pair have both bound to said protein;
f) elongating one of the reactive oligonucleotides by isothermal amplification using the circularized DNA molecule as template, thereby creating a localized amplification product;
g) detecting the presence and quantity of said protein using a detection oligonucleotide complementary to the amplification product;
wherein the binding between the proximity probe pairs and the protein as well as the detection are performed directly in an electrophoretic gel, or wherein the sample containing said protein is first separated using gel electrophoresis and then transferred from the electrophoretic gel onto a suitable blotting membrane, such as a nitrocellulose or PVDF membrane, before binding and detection with the proximity probes.

In certain embodiments, the binding moieties are antibodies and the antibodies each bind to the protein via the aid of one or two primary antibodies having direct binding specificity for the protein, and the binding moieties are directed against the Fc portion or conjugated haptens of the further antibody/antibodies.

In other embodiments, the binding moieties of the proximity probes have direct specificity for epitopes of the protein and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

In still other embodiments, the isothermal amplification is rolling circle amplification. Preferably, the rolling circle amplification is performed using Phi29 DNA polymerase.

In some embodiments, the sample is a homogenized tissue or cell lysate, a body fluid, or cell culture supernatant.

In the method of the invention, the binding between the proximity probes and the protein as well as the detection are performed directly in the electrophoretic gel. In methods described herein, the sample may be directly spotted onto a suitable membrane, such as nitrocellulose or PVDF without separation before binding and detection with the proximity probes.

In certain embodiments, the detection oligonucleotides are fluorescently labelled and the detection is performed by fluorescence read-out. In certain other embodiments, the detection oligonucleotides are labeled with an HRP enzyme and the detection is performed by ECL read-out.

In a particular embodiment of the invention, it is provided a method for the multiplexed detection of two or more target proteins in a sample in an electrophoretic gel or on a blotting membrane, wherein:
i) in step (b), a proximity probe pair is provided for each of the two or more target proteins;
ii) in step (d) a splint oligonucleotide and a backbone oligonucleotide is provided for each of the two or more target proteins;
iii) in step (f) a target-specific localized amplification product is created from different proximity probe pairs and each localised amplification product contains copies of a target specific sequence region; and
iv) in step (g) a unique detection oligonucleotide complimentary to the target specific sequence region of each of the localised amplification products is provided, wherein for each amplification product each such detection oligonucleotide
   islabelled with one or more different fluorescent dyes and defined an unique ratios of differently labelled detection oligonucleotides are pre-mixed to product a detection oligonucleotide mixture for each target protein, wherein each mixture contains a defined ratio of differently labelled detection oligonucleotides such that during detection, a unique signal ratio is associated with the amplification product for each protein, and wherein the mixtures are pooled and the complete set of detection oligonucleotide mixtures is added to the electrophoretic gel or blotting membrane;
and said method further comprises:
h) detecting the signal from each label for each of the two or more different labels;
i) generating a signal gain calibration for the different labels using a predefined standard sample present at a known and spatially separated location in or on the gel or membrane;
j) calculating the ratio of labels for each location using the signal gain calibration and detecting the target protein of interest based on the detection of the unique signal ratios.

In this embodiment, the method comprises: for each of the target proteins, providing one splint oligonucleotide and one backbone oligonucleotide which are complementary to the reactive oligonucleotides, thus a circularized DNA molecule is formed through hybridization and ligation of these oligonucleotides, provided both proximity probes bind to the same protein in proximity.

In certain embodiments, the binding moieties are antibodies and the antibodies each bind to the protein via the aid of one or two further antibody/antibodies having direct binding specificity for the protein, and wherein the binding moieties are directed against the Fc portion or conjugated haptens of the further antibody/antibodies.

In other embodiments, the isothermal amplification is rolling circle amplification. Preferably, the isothermal amplification is performed using Phi29 DNA polymerase.

In still other embodiments, the binding moieties of the proximity probes have direct specificity for the protein and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

In some embodiments, the sample is a homogenized tissue or cell lysate, a body fluid, or cell culture supernatant.

In certain embodiments, the binding between the proximity probes and the bio molecular feature as well as the detection are performed directly in an electrophoretic gel.

In other embodiments, the detecting step is performed by taking multiple scans/images at different excitation wavelength/emission filter combinations. In still other embodiments, the detecting step is performed by multi-spectral imaging (i.e. by recoding complete emission spectra in each pixel).

Also disclosed is a kit for proximity ligation assay based Western blot type of analysis. The kit comprises a proximity probe pair, each probe comprising a binding moiety with affinity for a different binding site on a bio molecular feature and an oligonucleotide acting as a reactive oligonucleotide, coupled thereto; a splint oligonucleotide and a backbone oligonucleotide which are complementary to the reactive oligonucleotide pair; a detection oligonucleotide; one or more optimized buffers, enzymes and protocols. In certain embodiments, the detection oligonucleotide is labeled. In other embodiments, the binding moieties are antibodies and the antibodies each bind to the bio molecular feature via the aid of one or two further antibody/antibodies having direct binding specificity for the bio molecular feature, and wherein the binding moieties are directed against the Fc portion or conjugated haptens of the further antibody/antibodies. In still other embodiments, the kit further comprises a DNA ligase and an enzyme for isothermal amplification, such as rolling circle amplification.

Also disclosed is a kit for multiplexed Western blot analysis of two or more target bio molecular features. The kit comprises, for each of the target bio molecular features: a proximity probe pair, each probe comprising a binding moiety with affinity for a different binding site on the bio molecular feature and an oligonucleotide acting as a reactive oligonucleotide, coupled thereto; a splint oligonucleotide and a backbone oligonucleotide which are complementary to the reactive oligonucleotide pair, thus a circularized DNA molecule can be formed and an amplification product can be generated during the reaction process provided both proximity probes bind to the same bio molecular feature in proximity; a detection oligonucleotide mixture based on a sequence which is complimentary to a unique detection sequence site on the amplification product; wherein the detection oligonucleotide mixture for each target bio molecular feature contains a defined ratio of species with identical sequence but labeled with different fluorescent dyes such that during detection, a unique signal ratio is associated with the amplification product for each substrate; the kit further includes a standard sample for calibration of fluorescent dye signal gains; one or more optimized buffers; and protocols. In one embodiment, the kit further comprises a DNA ligase and an enzyme for isothermal amplification, such as rolling circle amplification. In another embodiment, the binding moieties are antibodies and the antibodies each bind to the bio molecular feature via the aid of one or two further antibody/antibodies having direct binding specificity for the substrate, and wherein the binding moieties are directed against the Fc portion or conjugated haptens of the further antibody/antibodies. In another embodiment, the binding moieties have direct specificity for the bio molecular feature and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

### Brief Description of the Drawings

Figure 1 shows a schematic overview of standard in-situ PLA implementations of prior art.
Figures 2A, 2B and 2C show proof of principle, showing increased signal intensity for PLA, compared to ECL™ Plex, using Dot blot and fluorescence detection.
Figures 3A and 3B show the increased signal intensity for tubulin in cell lysates using PLA Western blot compared to traditional Western blotting with chemiluminescence detection.
Figures 4A and 4B show the increased specificity for dual recognition PLA Western compared to either traditional Western or single recognition PLA Western of both tubulin and Villin protein targets with chemiluminescence detection, in a cell lysate sample.
Figures 5A and 5B show direct single signal detection of protein modification with PLA Western compared to traditional Western blotting using chemiluminescence detection.
Figure 6 show an overview of the multiplexing design using ratio-mixed fluorescent detection probes.
Figure 7 shows an example of the multiplexing design.
Figure 8 shows resolution of ratio-mixed fluorescent dyes using a fluorescent scanner.

### Detailed Description of the Invention

### In Situ PLA Western Blot

Disclosed herein are methods for Western blot applications. In particular, it is provided a procedure for using PLA in Western blot applications. For the first time the Applicants have shown that PLA can be used in a Western blot application.

The detection scheme is based on *in-situ* PLA. Here, two PLA probes (oligo-conjugated antibodies or other affinity reagents) are used to probe the Western blot or Dot blot membrane (or the electrophoretic gel, in case of In-gel Western applications) for binding to two distinct protein epitopes (primary detection format) or two distinct primary antibody epitopes (for detection of a protein, protein modification or protein-protein interaction). If the two PLA-probes bind in proximity of each other they can prime the creation of a circularized DNA molecule. This is achieved by addition of a backbone oligo and a splint oligo (that are complementary to the PLA-probe oligos) and a ligase. The sequences are designed such that in the next step the circularized DNA molecule is bound only to one of the PLA probes. A DNA polymerase capable of strand displacement amplification (e.g., Phi29 DNA polymerase) and nucleotides are added and the PLA probe oligo is elongated via rolling circle amplification. In the final step the localized RCPs (rolling circle products) are detected via detection oligos either conjugated to horseradish peroxidase HRP for ECL read-out or to a fluorescent dye for fluorescent read-out (Figure 1).

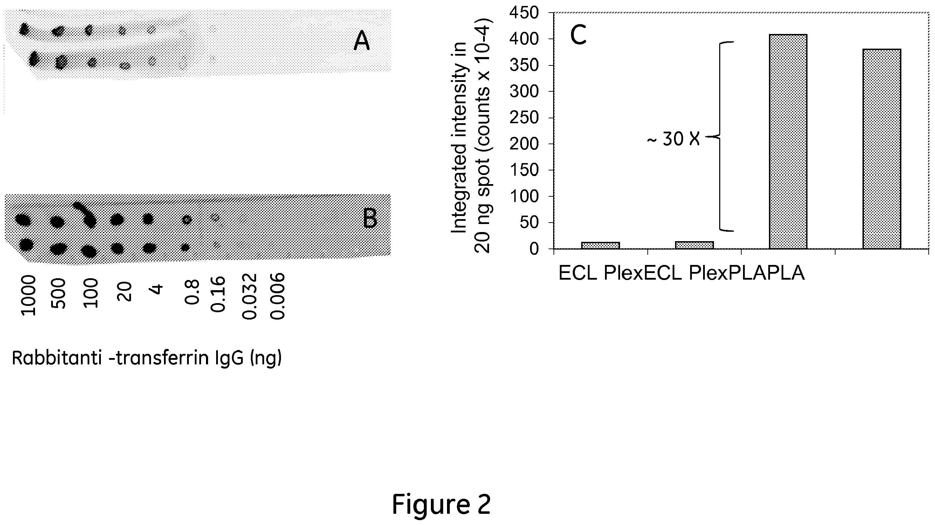

As proof of principle, the sensitivity of PLA was investigated using dot blot. For comparison, parallel experiments with ECL™ Plex detection have been done. Figure 2 shows signal intensity of the PLA method, compared to ECL™ Plex, using CY™5 detection, of a dilution series of rabbit anti-transferrin antibody. The results show approximately 30 fold increase in signal intensity for PLA detection compared to ECL™ Plex detection.

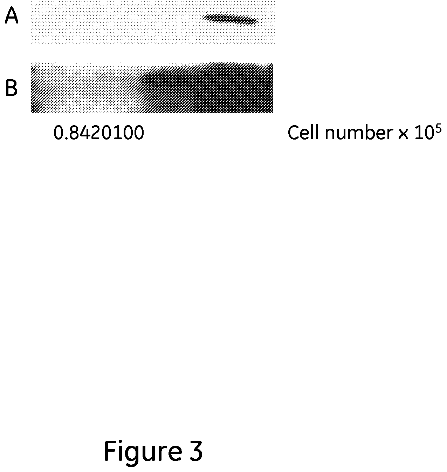

The sensitivity of PLA was shown by increased signal intensity in Western blot assays. A cell lysate dilution series was analyzed by Western blot, using chemiluminescence detection. Endogenous tubulin was detected by primary mouse anti-tubulin and secondary HRP conjugated antibody and ECL or PLA HRP/ECL detection. A 25-fold increase in signal intensity was observed for PLA (Figure 3).

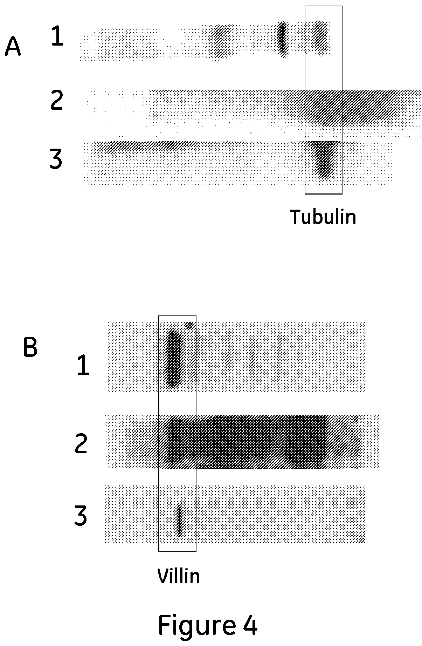

The specificity can be improved with removal of unspecific detection caused by poor quality primary antibodies. Using poor quality antibodies alone (single detection), give unspecific detection of protein bands whereas combining two poor quality primary antibodies using dual recognition PLA Western gives increased specificity in detection. Increased specificity was achieved for tubulin detection using dual recognition PLA Western compared to traditional ECL Western detection (Figure 4A). Specificity of Villin detection was highly improved using dual recognition PLA Western, compared to single recognition PLA Western using a liver tissue sample (Figure 4B).

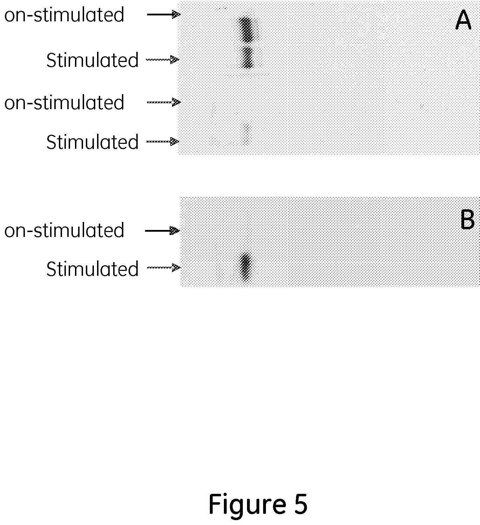

To assign a phosphorylation to a particular protein in current Western blot assays, signals from both a protein specific antibody and a phospho-epitope specific antibody need to be detected as two overlapping signals detected in separate channels (fluorescence), in separate lanes or blots (chemiluminescence). Using PLA Western, improved detection possibilities can be achieved by combining the two antibodies to generate a single signal if they bind in proximity (on the same protein). Detection of phosphorylated PDGFRβ with a single signal using PLA Western is achieved compared to traditional Western, where detection of two signals is needed (total protein and phosphorylated protein, see Figure 5).

Also disclosed is a kit for Western blot analysis, which kit comprises a proximity probe pair each probe comprising a binding moiety with affinity for a different binding site on a bio molecular feature (protein, protein complex or a specifically modified protein such as a phosphorylated protein) and an oligonucleotide acting as a reactive functionality (reactive oligonucleotide), coupled thereto; a splint oligonucleotide and a backbone oligonucleotide which are complementary to the reactive oligonucleotide; a detection oligonucleotide, one or more optimized buffers, and protocols. Optionally, the detection oligonucleotide is labeled. Optionally, the kit further comprises a DNA ligase and an enzyme for isothermal amplification, such as rolling circle amplification. As an example, Phi29 DNA polymerase is included as the enzyme for isothermal amplification.

In certain embodiments, the binding moieties are antibodies and the antibodies each bind to the bio molecular feature via the aid of one or two further antibody/antibodies having direct binding specificity for the bio molecular feature, and wherein the binding moieties are directed against the Fc portion and/or conjugated haptens of the further antibody/antibodies.

In other embodiments, the binding moieties have direct specificity for epitopes of the bio molecular feature and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

The use of *in-situ* PLA (proximity ligation assay) technology for Western blotting applications has been shown here to greatly increase the sensitivity and at the same time improve the specificity by the optional requirement of two proximal binding events at the primary level. For Western blotting, the benefit of increased sensitivity with the possibility to detect low abundant proteins in small amounts of samples cannot be overstated. For example, analysis of scarce samples (isolated stem cells/primary cells, xenograft aspirates and biopsies) not amendable to Western blot analysis previously is now within reach. Further, the increased sensitivity potentially enables improved In-gel Western blot analysis (detection performed in a near-surface volume of electrophoretic gels), thereby speeding up analysis and removing potential variation introduced during the blotting step.

Via the use of dual recognition events at the primary level, the specificity is highly increased. Due to this high specificity using dual recognition detection it would be possible in some applications to omit the electrophoresis size separation step before protein analysis and directly spot the sample on a membrane (Dot blot). By using dual recognition, only the target bio molecular feature will be detected and unspecific signals will not be generated. Traditionally, a size separation and molecular weight confirmation is needed to avoid the risk of unspecific detection at the migration position of the target protein ensuring analysis of true signals. The high specificity using dual recognition can also be exploited to extend the range of usable antibodies (and thereby targets) or to design unique assays detecting protein specific posttranslational modifications (such as phosphorylations, glycosylations or acetylations) or stable protein interactions (using native gel electrophoresis conditions) with high sensitivity and specificity.

### Multiplex of in situ PLA Western Blot

Based on the successful application of *in-situ* PLA in Western blot, another aspect of the disclosure shows a method for controlling/building localized fluorescent bar-codes based on combinations of target specific RCPs and fluorophore labeled detection oligonucleotides. This enables a procedure for increasing the obtainable multiplexing-level in Western blots without the need of a high number of fluorophores or the use of stripping methodologies or other repeated probing strategies. In addition the high sensitivity and unique detection specificity offered by PLA Western blot can thus be exploited for multiple targets at once, thereby further extending the amount and type of information that can be extracted from scarce samples (isolated stem cells/primary cells, xenograft aspirates and biopsies).

The following features of *in-situ* PLA are essential to this aspect of the disclosure:
(1) Fluorophores are administered via detection oligonucleotides. In contrast to antibody labeling this enables precise control over the number of fluorophore molecules per reagent (i.e. one).
(2) A large number of target sequence repeats (∼1000) are present (in target specific RCPs) locally for each specifically detected antigen. Hence, variation in actual fluorophore ratios for individual RCPs, due to statistical effects during hybridization, can be kept low.

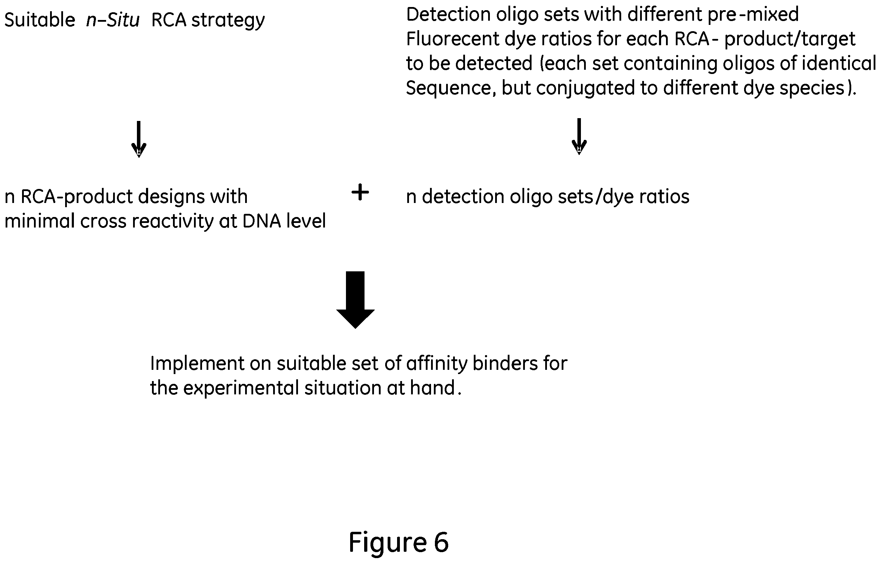

Thus, one embodiment of the disclosure provides a multiplexed *in situ* PLA-based Western blot method, using unique bar-codes based on defined ratios of two (or more) fluorophores in target specific RCPs (Figure 6). In this method, oligonucleotide sets (two oligos for creation of PLA probes via conjugation to affinity binders, plus one backbone and one splint oligo) for the number of targets to be detected is designed for minimal cross-reactivity during proximity ligation and RCA. Hence, using suitable affinity reagents, target specific RCPs can be generated during reactions on a Western blot membrane. Further, each target specific RCP is designed to contain amplified copies of a target specific sequence region which can promote hybridization to a unique detection oligonucleotide sequence. For each RCP a sequence complementary to the unique detection sequence is produced. Each such detection oligonucleotide sequence is conjugated to one or more different fluorescent dyes (in case of multiple dyes, different aliquots of unlabeled oligonucleotides are conjugated to single dyes). For each target bio molecular feature /RCP to be detected, defined and unique ratios of the differently labeled detection oligonucleotides (all having identical sequence) are pre-mixed (one mixture for each target bio molecular feature /unique RCP). The complete set of detection oligonucleotide mixtures are added to the reaction containing the membrane, and the dye ratios are transferred to localized target specific RCPs via sequence specific hybridization. A fluorescent imager or scanner is used to generate multiple images (one with optimal settings for each dye included) of the membrane. De-coding is dependent on proper calibration of signal gains for the different dyes included. For the purpose of image acquisition equipment calibration, a well-defined standard sample (probed using one of the RCP designs used for unknown samples and an equimolar mixture of all the dyes included in the experiment) is present at a known and spatially separated location on the membrane. Following calibration, measured dye ratios for different locations on the membrane can be de-coded. Quantitative analysis is then performed using optimal images for each target.

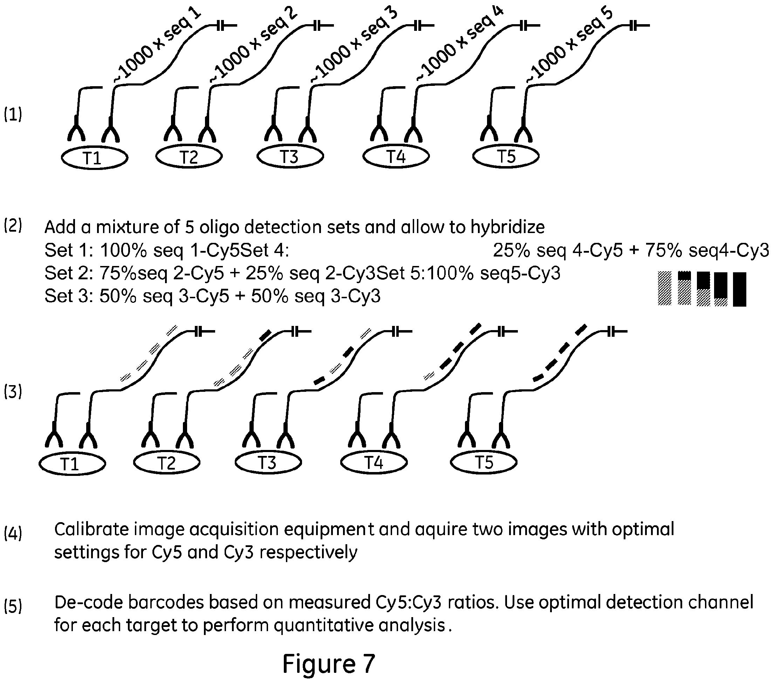

Figure 7 provides an example of a 5-plex PLA Western blot using only CY™3 and CY™5, with localized "bar-codes":
(1) Each target bio molecular feature to be analyses is assigned a bar-code based on a defined ratio of CY™5 to CY™3 (only CY™5, 3:1, 1:1, 1:3 and only CY™3). Five PLA reagent sets (each containing two PLA probes and corresponding backbone-, splint- and detection oligonucleotides) are designed according to the scheme outlined in the general description. Each set recognizes a unique target bio molecular feature on a Western membrane and generates target specific RCPs, with minimal interference from other sets, following proximity ligation and RCA.
(2) Aliqout(s) of each detection oligonucleotide are labeled with CY™5, CY™3 or both and then mixed in the predetermined CY™5/ CY™3 ratio. Thus the group of five detection oligonucleotide sequences are present in the following mixtures: (1) 100% CY™5; (2) 3:1 of CY™5: CY™3; (3) 1:1 of CY™5: CY™3; (4) 1:3 of CY™5: CY™3; and (5) 100% CY™3; respectively.
(3) Detection oligonucleotides for the five target bio molecular features are mixed together, added to the membrane and thereby transferred to corresponding local RCPs via sequence-specific hybridization.
(4) A suitable image acquisition equipment is used to generate images with optimal settings for CY™5 and CY™3, respectively. Calibration of CY™5 to CY™3 signal gain is achieved via the procedure outlined in the general description (using a spatially separated standard sample).
(5) The "barcodes" are decoded based on the signal gain calibration and measured CY™5:CY™3 ratios. Furthermore, optimal detection channels are used for each target to perform quantitative analysis (channel/image with highest signal to noise chosen for each target/barcode).

Thus, as shown in Figure 7, following proximity ligation and RCA each unique target bio molecular feature (e.g., protein, protein modification or protein complex) can be detected using an oligonucleotide the sequence of which corresponds to a complimentary sequence of the corresponding RCP. The oligonucleotides are labeled with either CY™5 or CY™3, but the combined oligonucleotides pool for each target has a predetermined ratio of CY™5 and CY™3. The oligonucleotide pool of oligonucleotides for each target has a ratio of CY™5 and CY™3 distinguishable from the ratios of other oligonucleotides pools. Thus, multiplexing is achieved. Although Figure 7 presents an example with two dyes, it could be advantageous in certain circumstances to use more than two dyes. The principle is similar nonetheless.

An optimized set of bar-code oligonucleotide sets as outlined above can be transferred to any desired set of antibodies (or other affinity reagents) and used in different assay set-ups. To reach really high multiplexing, oligonucleotide conjugation needs to be performed at the primary binder level due to the limited number of different sources (species) of antibodies available. However, the principle can also be applied to secondary detection formats. For example to design a 5-plex secondary kit using only two fluorophores.

Also disclosed is a kit for multiplexed Western blot analysis of two or more target bio molecular features, comprising: for each of the target substrates, a proximity probe pair, each probe comprising a binding moiety with affinity for a different binding site on the bio molecular feature and an oligonucleotide acting as a reactive functionality (reactive oligonucleotide), coupled thereto; a splint oligonucleotide and backbone oligonucleotide which are complementary to the reactive oligonucleotides, thus a circularized DNA molecule can be formed and an amplification product can be generated during the reaction process, provided both proximity probes bind to the same bio molecular feature in proximity; a detection oligonucleotide mixture based on a sequence which is complimentary to a unique detection sequence site on the amplification product; wherein the detection oligonucleotide mixture for each target bio molecular feature contains a defined ratio of species with identical sequence but labeled with different fluorescent dyes such that during detection, a unique signal ratio is associated with the amplification product for each bio molecular feature, the kit further includes a standard sample for calibration of fluorescent dye signal gains, one or more optimized buffers, and protocols. Optionally, the kit further comprises a DNA ligase and an enzyme for isothermal amplification, such as rolling circle amplification. As an example, Phi29 DNA polymerase is included as the enzyme for isothermal amplification.

In certain embodiments, the binding moieties are antibodies and the antibodies each bind to the bio molecular feature via the aid of one or two further antibody/antibodies having direct binding specificity for the bio molecular feature, and wherein the binding moieties are directed against the Fc portion and/or conjugated haptens of the further antibody/antibodies.

In other embodiments, the binding moieties have direct specificity for the bio molecular feature and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

### Examples

### Experimental

### Electrophoresis and transfer:

Standard electrophoresis (miniVE Vertical Electrophoresis System, GE Healthcare) and wet transfer (TE 22 Mini Tank Transfer Unit, GE Healthcare) protocols were used according to manufactures instructions.

### Protein samples

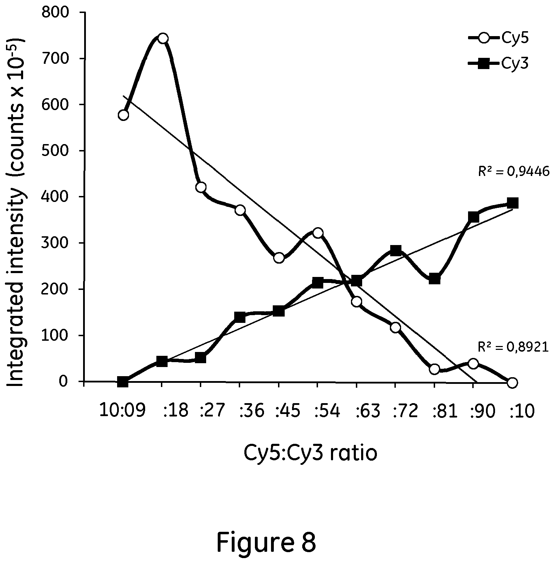

Human fibroblast cell lysate (cell line BJh-TERT).
Rabbit anti-transferrin antibody (Sigma) Dot blot experiment (Figure 2).
Liver tissue extract.
ECL Plex anti-mouse CY™5 and CY™3 for Dot blot for ratio-mix experiments (Figure 8).

### Primary antibodies:

Rabbit anti-transferrin antibody (Sigma, 1:750 dilution).
Mouse anti-β-tubulin antibody (Sigma, 1:2000 dilution).
Rabbit anti-tubulin, whole antiserum (Sigma, 1:200 dilution).
Chicken anti-TUBB2A antibody (Sigma, 1:3000 dilution).
Rabbit anti-PDGF receptor β (28E1), (Cell Signaling, 1:1300 dilution).
Mouse anti-Phospho-PDGF Receptor b (Tyr751) (88H8) (Cell Signaling, 1:1300 dilution).

Rabbit anti-Villin 6884.
Rabbit anti-Villin 6885.

### Traditional Western blotting:

Tris-Glycine pre-cast gel (12 % 15 well or 7.5% polyacryl gel, 26-well), 2%(w/v) ECL™ Advance Blocking Agent in TBS 0.1 % TWEEN™, HYBOND™-LFP PVDF membrane HYBOND™-LFP, 0.1% TBS TWEEN™ 20 or TBS wash buffers and Amersham ECL™, ECL™ Plus (anti mouse, rabbit or chicken HRP secondary antibody) or ECL™ Plex (anti-rabbit CY5 secondary antibody) Western blotting detection systems. Probing, washing and detection were performed according to manufactures instructions.

### PLA Western blotting:

Tris-Glycine pre-cast gel (12 % 15 well or 7.5% polyacryl gel, 26-well), 3% (w/v) BSA blocking agent in TBS, 0.1% TWEEN™ 20, 100µg/ml salmon sperm DNA.
PLA buffer: TBS buffer, 0.5 mg/ml BSA, 5 µg/ml salmon sperm DNA, 5 mM EDTA, 0.05%
TWEEN™ 20. Oligo ligation/hybrid buffer: 150 / 190 mM NaCl, 0.25 mg/ml BSA, 0.05% TWEEN™ 20, 0.5 mM ATP.
T4 ligase buffer (-DTT): 10 mM Tris-Ac, 10 mM MgAc, 50 mM KAc.
RCA buffer: 0.125 mM each of dNTPs, 0.25 mg/ml BSA, 0.05% TWEEN™ 20.
phi29 polymerase buffer: 50 mM Tris-HCl, 10 mM MgCl2, 10 mM (NH4)2SO4, PH 7.5. Detection buffer: 2×SSC, 0.25 mg/ml BSA, 5 µg/ml salmon sperm DNA, 0.05% TWEEN™ 20.
PLA probe preserving buffer: 1×PBS, 0.05% NaN₃, 0.2 mg/ml BSA.
Anti-mouse, rabbit or chicken secondary PLA probe antibodies and oligo sequences:
   PLA probe sequence 1 (SEQ ID NO: 1): antibody-5'- AAA AAA AAA ATA TGA CAG AAC-TA GAC ACT CTT - 3' conjugate.
   PLA probe sequence 2 (SEQ ID NO: 2): antibody-5' - AAA AAA AAA AGA CGC TAA TAG TTA AGA CGC TTU UU - 3' conjugate.
   Backbone oligo (SEQ ID NO: 3): 5' - CTA TTA GCG TCC AGT GAA TGC GAG TCC GTC
   TAA GAG AGT AGT ACA GCA GCC GTC AAG AGT GTC TA - 3'
   Splint oligo (SEQ ID NO: 4): 5' - GTT CTG TCA TAT TTA AGC GTC TTA A - 3'
   Detection probe sequence (SEQ ID NO: 5): CAG TGA ATG CGA GTC CGT CT
   FITC- detection probe (SEQ ID NO: 6): FITC - AAA AAA CAG TGA ATG CGA GTC CGT CT.
   HRP- detection probe (SEQ ID NO: 7): HRP - AAA AAA AAA CAG TGA ATG CGA GTC
   CGT CT. (HRP with the diameter of 5 nm, oligo length about 9.5 nm)
   CY™5- detection probe (SEQ ID NO: 6): CY™5 - AAA AAA CAG TGA ATG CGA GTC CGT CT.
   CY™3- detection probe (SEQ ID NO: 6): CY™3 - AAA AAA CAG TGA ATG CGA GTC CGT CT.

### PLA Western probing protocol:

Incubate primary antibody with gentle orbital rotation in a 5-ml plastic tube chamber at 4°C overnight. Rinse the membrane once in a plastic container, and then wash 3 × 9 min with 10ml TBST in a 15-ml tube by gentle rocking at room temperature. Incubate PLA minus and PLA plus probes in 1X PLA buffer (Olink) containing 0.5 % goat serum gentle orbital rotation in a 5-ml plastic tube chamber at room temperature for 1 hour (total volume 1700 µl). Briefly rinse membrane twice with TBS-T. Wash with excess TBS-T, 2×9 min using 15ml tube with 10ml washing buffer by gentle rocking at room temperature. Perform backbone/splint oligos hybridization and ligation by incubating 90nM backbone and splint oligos, 0.05U/µl T4 ligase in 1X ligation/hybridization buffer(Olink) and 1X ligase buffer (without DTT)(Olink) at 37°C for 40 minutes during orbital rotation in a 5ml plastic tube chamber. Briefly rinse once in TBS-T.

Wash with 10ml TBS-T in a 15-ml tube by rocking, 2×6 minutes. Perform rolling circle amplification (RCA) by incubating 0.08 U/µl Phi29 DNA polymerase in 1X Phi29 polymerase buffer and 1X RCA buffer (Olink) at 37°C for 1 hour during orbital rotation in a 5 ml plastic tube chamber. Briefly rinse once in TBS-T. Wash with 10ml TBST in a 15ml tube by gentle rocking once for 6 minutes. Hybridize HRP- or CY™ dye labeled detection probe to RCA product by incubating 5 nM (ECL-readout) or 15 nM (fluorescent-readout) detection probe in 1X detection buffer (Olink) containing 2.5% formamide at 37°C for 30 minutes during orbital rotation in a 5ml plastic tube chamber. Briefly rinse twice in TBS-T. Wash with excess TBS-T, 3×9 minutes in a 15ml tube with 10ml washing buffer by gentle rocking at room temperature.

### Detection and image analysis:

Chemiluminescent (ECL) detection was made by film exposure followed by digitalization of the film using ImageScanner III (GE Healthcare) and fluorescent CY™5 signals were captured by using a fluorescent imager (TYPHOON™ 9410, GE Healthcare). Digital images were then analyzed by using ImageQuant™ TL image analysis software (GE Healthcare) and the signals were quantified.

### Results

Dot blot membranes with a dilution series ranging between 100 ng and 6 pg rabbit anti-transferrin antibody was probed with secondary ECL™ Plex CY™5 antibody (Figure 2A) or PLA CY™5 probe (Figure 2B). Signal intensity for CY™5 signals was increased 30 fold for PLA Western compared to ECL™ Plex fluorescent Western blotting using the same intensity setting during scanning with TYPHOON™ Imager (Figure 2C). Thus, increased fluorescent signal amplification was observed with PLA Western dot blot.

Western blot membranes with a dilution series of human fibroblast cell lysate ranging between 1 x 10⁵ to 160 cells were probed with mouse anti tubulin primary antibody and anti-mouse secondary HRP antibody (Figure 3A) or PLA HRP probe (Figure 3B). The membranes were incubated with ECL reagent and exposed to film for 3 minutes simultaneously (Figure 3). Increased chemiluminscence signal amplification and improved detection limit was observed with PLA Western.

Human fibroblast cell lysate was applied to Western blotting and the membranes were probed with rabbit anti-tubulin (Figure 4A, panel 1) or chicken anti-tubulin (Figure 4A, panel 2) and detected by traditional ECL Western blotting. Both these anti-tubulin primary antibodies showed unspecific protein detection when used separately by traditional ECL Western blotting. When tubulin instead was detected by dual recognition PLA Western (ECL read-out) using a combination of rabbit anti-tubulin and chicken anti-tubulin (Figure 4A, panel 3) the unspecific detection seen in panel 1 and 2 was removed. In another experiment, tissue samples from liver were applied to Western blotting. The membrane was probed with single PLA Western recognition using either 6884 (Figure 4B, panel 1) or 6885 (Figure 4B, panel 2) rabbit anti-Villin primary antibody showing unspecific detection of protein bands. When the membrane was probed with dual PLA Western recognition using both anti-Villin 6884 and 6885 primary antibody (Figure 4B, panel 3) the unspecific detection was removed. Increased specificity was observed using dual recognition PLA Western compared to single recognition PLA Western (Figure 4).

Non-stimulated and stimulated human fibroblast cells were applied to Western blotting. One membrane was probed with rabbit anti-PDGFRβ and mouse anti-phoshorylated PDGFRβ showing receptor signal and weak phosphorylation signal separately using traditional ECL Western blotting (Figure 5A). The other membrane was probed using dual recognition PLA Western using rabbit anti-PDGFRβ and mouse anti- phoshorylated PDGFRβ showing a single enhanced signal for phosphorylated receptor (Figure 5B). Single detection of protein modification was demonstrated with PLA Western.

To investigate how many ratios of Cy5:Cy3 that could be resolved using a fluorescent Imager, ratios ranging from 10:0 to 0:10 (eleven steps, 10 % difference) of ECL Plex anti-mouse Cy5 and Cy3 was mixed and spotted onto a PVDF membrane and scanned at optimal intensity setting (strongest signal just below saturation) in each channel. The results show that at least 6 different ratios of Cy5:Cy3 could be resolved using the Typhoon Imager (Figure 8).

## Claims

1. A method for detecting a protein in a sample, wherein said protein has been separated in an electrophoretic gel or on a blotting membrane, said method comprising:
a) providing a said gel or a said membrane containing a said protein from said sample;
b) providing a proximity probe pair, each probe comprising a binding moiety with affinity for a different binding site on said protein and a reactive oligonucleotide, coupled thereto;
c) binding said proximity probes to their respective binding sites on said protein through the binding moiety;
d) adding a splint oligonucleotide and a backbone oligonucleotide which are complementary to the reactive oligonucleotide pair, and allowing them to hybridize to the reactive oligonucleotides, such that when said probes in said proximity probe pair have both bound to said protein the respective ends of the splint oligonucleotide are hybridised directly in contact with the ends of the backbone oligonucleotide;
e) ligating, using a DNA ligase, the hybridized backbone and splint oligonucleotides to create a circularized DNA molecule wherein said circularized DNA molecule is formed from the backbone and splint oligonucleotides only when said probes in said proximity probe pair have both bound to said protein;
f) elongating one of the reactive oligonucleotides by isothermal amplification using the circularized DNA molecule as template, thereby creating a localized amplification product; and
g) detecting the presence and quantity of said protein using a detection oligonucleotide complementary to the amplification product;
wherein the binding between the proximity probe pairs and the protein as well as the detection are performed directly in an electrophoretic gel, or wherein the sample containing said protein is first separated using gel electrophoresis and then transferred from the electrophoretic gel onto a suitable blotting membrane, such as a nitrocellulose or PVDF membrane, before binding and detection with the proximity probes.

2. The method of claim 1, wherein said detection oligonucleotides are (i) fluorescently labeled and the detection is performed by fluorescence read-out, or (ii) labeled with an HRP enzyme and the detection is performed by ECL read-out.

3. The method of claim 1 for the multiplexed detection of two or more target proteins in a sample in an electrophoretic gel or on a blotting membrane, wherein:
i) in step (b) a proximity probe pair is provided for each of the two or more target proteins ;
ii) in step (d) a splint oligonucleotide and a backbone oligonucleotide is provided for each of the two or more target proteins ;
iii) in step (f) a target-specific localised amplification product is created from different proximity probe pairs and each localised amplification product contains copies of a target specific sequence region; and
iv) in step (g) a unique detection oligonucleotide complementary to the target specific sequence region of each of the localised amplification products is provided, wherein for each amplification product each such detection oligonucleotide is labelled with one or more different fluorescent dyes and defined and unique ratios of differently labelled detection oligonucleotides are pre-mixed to produce a detection oligonucleotide mixture for each target protein, wherein each mixture contains a defined ratio of differently labelled detection oligonucleotides such that during detection a unique signal ratio is associated with the amplification product for each protein, and wherein the mixtures are pooled and the complete set of detection oligonucleotide mixtures is added to the electrophoretic gel or blotting membrane;
and said method further comprises:
h) detecting the signal from each label for each of the two or more different labels;
i) generating a signal gain calibration for the different labels using a predefined standard sample present at a known and spatially separated location in or on the gel or membrane; and
j) calculating the ratio of labels for each location using said signal gain calibration and detecting the target protein of interest based on the detection of the unique signal ratios.

4. The method of any one of claims 1 to 3, wherein said protein is a protein complex or a modified protein such as a phosphorylated protein.

5. The method of any one of claims 1 to 4, wherein the binding moieties are antibodies and said antibodies each bind to said protein via the aid of one or two further antibody/antibodies having direct binding specificity for the protein, and wherein the binding moieties are directed against the Fc portion or conjugated haptens of the further antibody/antibodies.

6. The method of any one of claims 1 to 5, wherein said isothermal amplification is rolling circle amplification, preferably wherein said isothermal amplification is performed using Phi29 DNA polymerase.

7. The method of any one of claims 1 to 6, wherein the binding moieties of the proximity probes have direct specificity for the protein and are selected from a protein, such as a monoclonal or polyclonal antibody, lectin, soluble cell surface receptor, combinatorially derived protein from phage display or ribosome display, peptide, carbohydrate, nucleic acid, such as an aptamer, or combinations thereof.

8. The method of any one of claims 1 to 7, wherein said sample is a homogenized tissue or cell lysate, a body fluid, or cell culture supernatant.

9. The method of any one of claims 3 to 8, wherein said detecting step is performed by taking multiple scans/images at different excitation wavelength/emission filter combinations, or by multi-spectral imaging (i.e. by recoding complete emission spectra in each pixel).

## Patentansprüche

1. Verfahren zum Erkennen eines Proteins in einer Probe, wobei das Protein in ein elektrophoretisches Gel oder auf eine Blotting-Membran abgesondert wurde, das Verfahren umfassend:
a) Vorsehen des Gels oder der Membran, die das Protein aus der Probe enthalten;
b) Vorsehen eines Näherungssondenpaars, wobei jede Sonde einen Bindungsanteil mit einer Affinität für eine unterschiedliche Bindungsstelle am Protein und ein reaktives Oligonukleotid umfasst, das daran gekoppelt ist;
c) Binden der Näherungssonden an ihren jeweiligen Bindungsstellen am Protein durch den Bindungsanteil;
d) Zusetzen eines Splint-Oligonukleotids und eines Backbone-Oligonukleotids, die komplementär zum reaktiven Oligonukleotidenpaar sind, und Ermöglichen, dass sie an den reaktiven Oligonukleotiden hybridisieren, sodass, wenn die Sonden in der Näherungssonde beide am Protein gebunden haben, jeweilige Enden des Splint-Oligonukleotids direkt in Kontakt mit den Enden des Backbone-Oligonukleotids hybridisiert sind;
e) Ligieren, unter Benutzung einer DNA-Ligase, des hybridisierten Backbone- und Splint-Oligonukleotids zum Schaffen eines zirkularisierten DNA-Moleküls, wobei das zirkularisierte DNA-Molekül nur dann aus dem Backbone- und Splint-Oligonukleotid ausgebildet wird, wenn die Sonden im Näherungssondenpaar beide an das Protein gebunden haben;
f) Verlängern von einem der reaktiven Oligonukleotide durch isothermische Vergrößerung unter Benutzung des zirkularisierten DNA-Moleküls als Vorlage, wodurch ein lokalisiertes Vergrößerungsprodukt geschaffen wird; und
g) Erkennen des Vorhandenseins und der Menge des Proteins unter Benutzung eines Erkennungsoligonukleotids, das zum Vergrößerungsprodukt komplementär ist; wobei sowie das Binden zwischen den Näherungssondenpaaren und dem Protein als auch die Erkennung direkt in einem elektrophoretischen Gel ausgeführt wird, oder wobei die Probe, die das Protein enthält, zunächst unter Anwendung von Gelelektrophorese abgesondert wird und dann aus dem elektrophoretischen Gel auf eine geeignete Blotting-Membran, wie etwa eine Nitrocellulose- oder PVDF-Membran, übertragen wird, vor dem Binden und der Erkennung mit den Näherungssonden.

2. Verfahren nach Anspruch 1, wobei die Erkennungsoligonukleotide (i) fluoreszent markiert werden und die Erkennung durch Fluoreszenzablesung ausgeführt wird, oder (ii) mit einem HRP-Enzym markiert werden und die Erkennung durch ECL-Ablesung ausgeführt wird.

3. Verfahren nach Anspruch 1 für die Multiplexerkennung von zwei oder mehr Zielproteinen in einer Probe in einem elektrophoretischen Gel oder auf einer Blotting-Membran, wobei:
i) in Schritt b) eine Näherungssonde für jedes der zwei oder mehr Zielproteine vorgesehen wird;
ii) in Schritt d) ein Splint-Oligonukleotid und ein Backbone-Oligonukleotid für jedes der zwei oder mehr Zielproteine vorgesehen wird;
iii) in Schritt f) ein zielspezifisches lokalisiertes Vergrößerungsprodukt aus verschiedenen Näherungsprobenpaaren geschaffen wird und jedes lokalisierte Vergrößerungsprodukt Kopien einer zielspezifischen Sequenzregion enthält;
iv) in Schritt g) ein einmaliges Erkennungsoligonukleotid, das zur zielspezifischen Sequenzregion von jedem der lokalisierten Vergrößerungsprodukte komplementär ist, vorgesehen wird, wobei für jedes Vergrößerungsprodukt jedes derartige Erkennungsoligonukleotid mit einem oder mehr verschiedenen fluoreszenten Farbstoffen markiert und definiert wird und einmalige Verhältnisse von verschiedenen markierten Erkennungsoligonukleotiden zum Erzeugen einer Erkennungsoligonukleotidmischung für jedes Zielprotein vorgemischt werden, wobei jede Mischung ein definiertes Verhältnis von verschieden markierten Erkennungsoligonukleotiden enthält, sodass während der Erkennung dem Vergrößerungsprodukt für jedes Protein ein einmaliges Signalverhältnis zugeordnet werden kann, und wobei die Mischungen zusammengefasst werden und der vollständige Satz von Erkennungsoligonukleotidmischungen dem elektrophoretischen Gel oder der Blotting-Membran zugesetzt wird;
und wobei das Verfahren ferner folgendes umfasst:
h) Erkennen des Signals von jeder Markierung für jede der zwei oder mehr verschiedenen Markierungen;
i) Erzeugen einer Signalverstärkungskalibrierung für die verschiedenen Markierungen unter Benutzung einer vordefinierten Standardprobe, die an einer bekannten und räumlich getrennten Stelle in oder auf dem Gel oder der Membran vorhanden ist;
j) Berechnen des Verhältnisses von Markierungen für jede Stelle unter Benutzung der Signalverstärkungskalibrierung und Erkennen des relevanten Zielproteins auf Grundlage der Erkennung der einmaligen Signalverhältnisse.

4. Verfahren nach einem der Ansprüche 1 bis 3, wobei das Protein ein Proteinkomplex oder ein modifiziertes Protein, wie etwa ein phosphoryliertes Protein, ist.

5. Verfahren nach einem der Ansprüche 1 bis 4, wobei die Bindungsanteile Antikörper sind und die Antikörper jeder über die Säure von einem oder zwei weiteren Antikörper/Antikörpern, die direkte Bindungsspezifität für das Protein aufweisen, an das Protein binden, und wobei die Bindungsanteile gegen den Fc-Abschnitt oder konjugierte Hapten des/der weiteren Antikörper/s gerichtet sind.

6. Verfahren nach einem der Ansprüche 1 bis 5, wobei die isothermische Vergrößerung "Rolling Circle"-Vergrößerung ist, vorzugsweise wobei die isothermische Vergrößerung unter Anwendung von Phi29-DNA-Polymerase durchgeführt wird.

7. Verfahren nach einem der Ansprüche 1 bis 6, wobei die Bindungsanteile der Näherungssonden direkte Spezifität für das Protein aufweisen und aus einem Protein, wie etwa einem monoklonalem oder polyklonalem Antikörper, Lectin, löslichem Zellenoberflächenrezeptor, kombinatorisch abgeleiteten Protein von Phage-Display oder Ribosom-Display, Peptid, Kohlenhydrat, Nucleinsäure, wie etwa einem Aptamer, oder Kombinationen davon ausgewählt sind.

8. Verfahren nach einem der Ansprüche 1 bis 7, wobei die Probe ein homogenisiertes Gewebe- oder Zelllysat, eine Körperflüssigkeit oder ein Zellkulturenüberstand ist.

9. Verfahren nach einem der Ansprüche 3 bis 8, wobei der Erkennungsschritt durch Abnehmen von mehreren Abtastungen/Bildern auf verschiedenen Erregungswellenlängen-/Emissionsfilterkombinationen oder durch Multispektralbildgebung (beispielsweise durch Aufzeichnen von kompletten Emissionsspektren in jedem Pixel) ausgeführt wird.

## Revendications

1. Procédé pour détecter une protéine dans un échantillon, dans lequel ladite protéine a été séparée dans un gel électrophorétique ou sur une membrane pour transfert, ledit procédé comprenant :
a) la fourniture d'un dit gel ou d'une dite membrane contenant une dite protéine provenant dudit échantillon ;
b) la fourniture d'un couple de sondes de proximité, chaque sonde comprenant une fraction de liaison avec affinité pour un site de liaison différent sur ladite protéine et un oligonucléotide réactif, couplé à cette dernière ;
c) la liaison desdites sondes de proximité à leurs sites de liaison respectifs sur ladite protéine par l'intermédiaire de la fraction de liaison ;
d) l'ajout d'un oligonucléotide épissure et d'un oligonucléotide épine dorsale qui sont complémentaires au couple d'oligonucléotides réactifs, et leur permettant de s'hybrider aux oligonucléotides réactifs, de sorte que lorsque lesdites sondes dans ledit couple de sondes de proximité sont toutes les deux liées à ladite protéine, les extrémités respectives de l'oligonucléotide épissure sont hybridées directement en contact avec les extrémités de l'oligonucléotide épine dorsale ;
e) la ligature, en utilisant une ADN ligase, des oligonucléotides de chaîne principale et d'attelle hybridés pour créer une molécule d'ADN circularisé dans laquelle ladite molécule d'ADN circularisé est formée à partir des oligonucléotides de chaîne principale et d'attelle seulement si lesdites sondes dans ledit couple de sondes de proximité sont toutes les deux liées à ladite protéine ;
f) l'allongement d'un des oligonucléotides réactifs par amplification isotherme en utilisant la molécule d'ADN circularisé comme matrice, créant de ce fait un produit d'amplification localisé ; et
g) la détection de la présence et de la quantité de ladite protéine en utilisant un oligonucléotide de détection complémentaire au produit d'amplification ;
dans lequel la liaison entre les couples de sondes de proximité et la protéine aussi bien que la détection sont effectuées directement dans un gel électrophorétique, ou dans lequel l'échantillon contenant ladite protéine est d'abord séparé en utilisant une électrophorèse sur gel et est transféré ensuite du gel électrophorétique sur une membrane de transfert appropriée, comme une membrane de nitrocellulose ou de PVDF, avant la liaison et la détection avec les sondes de proximité.

2. Procédé selon la revendication 1, dans lequel lesdits oligonucléotides de détection sont (i) marqués de manière fluorescente et la détection est effectuée par extraction de fluorescence, ou (ii) marqués d'une enzyme HRP et la détection est effectuée par extraction ECL.

3. Procédé selon la revendication 1, pour la détection multiplexée de deux ou plusieurs protéines cibles dans un échantillon dans un gel électrophorétique ou sur une membrane de transfert, dans lequel :
i) dans l'étape (b) un couple de sondes de proximité est prévu pour chacune des deux ou plusieurs protéines cibles ;
ii) dans l'étape (d) un oligonucléotide épissure et un oligonucléotide épine dorsale sont prévus pour chacune des deux ou plusieurs protéines cibles ;
iii) dans l'étape (f) un produit d'amplification localisé spécifique à une cible est créé à partir de différents couples de sonde de proximité et chaque produit d'amplification localisé contient des copies d'une région de séquence spécifique à une cible ; et
iv) dans l'étape (g) un oligonucléotide de détection unique complémentaire à la région de séquence spécifique à une cible de chacun des produits d'amplification localisés est prévu, dans lequel pour chaque produit d'amplification chaque tel oligonucléotide de détection est marqué d'un ou plusieurs colorants fluorescents différents et des rapports définis et uniques d'oligonucléotides de détection différemment étiquetés sont prémélangés pour produire un mélange d'oligonucléotides de détection pour chaque protéine cible, dans lequel chaque mélange contient un rapport défini d'oligonucléotides de détection différemment étiquetés de sorte que pendant la détection un rapport de signal unique est associé au produit d'amplification pour chaque protéine, et dans lequel les mélanges sont réunis et le jeu complet de mélanges d'oligonucléotides de détection est ajouté au gel électrophorétique ou à la membrane de transfert ;
et ledit procédé comprend en outre :
h) la détection du signal provenant de chaque étiquette pour chacune des deux ou plusieurs étiquettes différentes ;
i) la production d'un étalonnage de gain de signal pour les différentes étiquettes en utilisant un échantillon standard prédéfini présent au niveau d'un emplacement connu et séparé dans l'espace dans ou sur le gel ou la membrane ; et
j) le calcul du rapport d'étiquettes pour chaque emplacement en utilisant ledit étalonnage de gain de signal et la détection de la protéine cible d'intérêt sur la base de la détection des rapports uniques de signal.

4. Procédé selon l'une quelconque des revendications 1 à 3, dans lequel ladite protéine est un complexe protéique ou une protéine modifiée comme une protéine phosphorylée.

5. Procédé selon l'une quelconque des revendications 1 à 4, dans lequel les fractions de liaison sont des anticorps et lesdits anticorps se lient chacun à ladite protéine à l'aide d'un ou deux anticorps supplémentaire(s) ayant une spécificité de liaison directe pour la protéine, et dans lequel les fractions de liaison sont dirigées contre la partie Fc ou les haptènes conjugués du ou des anticorps supplémentaire(s).

6. Procédé selon l'une quelconque des revendications 1 à 5, dans lequel ladite amplification isotherme est une amplification par cercle roulant, de préférence dans lequel ladite amplification isotherme est effectuée en utilisant une polymérase d'ADN Phi29.

7. Procédé selon l'une quelconque des revendications 1 à 6, dans lequel les fractions de liaison des sondes de proximité ont une spécificité directe pour la protéine et sont sélectionnées à partir d'une protéine, comme un anticorps monoclonal ou polyclonal, la lectine, un récepteur de surface de cellule soluble, une protéine obtenue de façon combinatoire à partir d'une présentation sur phages ou affichage de ribosome, un peptide, un hydrate de carbone, un acide nucléique, comme un aptamère, ou des combinaisons de ceux-ci.

8. Procédé selon l'une quelconque des revendications 1 à 7, dans lequel ledit échantillon est un tissu homogénéisé ou un lysat cellulaire, un fluide corporel, ou un surnageant de culture cellulaire.

9. Procédé selon l'une quelconque des revendications 3 à 8, dans lequel ladite étape de détection est effectuée en prenant de multiples numérisations / images à des combinaisons de filtre de longueur d'onde / d'émission d'excitation différentes, ou par une imagerie multispectrale (c'est-à-dire en recodant des spectres d'émission complets dans chaque pixel).


### SEQUENCE LISTING

<110> GE HEALTHCARE BIO-SCIENCES AB
   LANDEGREN, Ulf
   HAGNER-MCWHIRTER, Asa
   IVANSSON, Daniel
<120> PROXIMITY LIGATION TECHNOLOGY FOR WESTERN BLOT APPLICATIONS
<130> PU1055
<140> PCT to be assigned
   <141> 2011-10-27
<150> US 61/407944
   <151> 2010-10-29
<150> US 61/411955
   <151> 2010-11-10
<160> 7
<170> PatentIn version 3.5
<210> 1
   <211> 32
   <212> DNA
   <213> Artificial Sequence
<220>
   <223> Synthetic Oligonucleotide
<400> 1
   aaaaaaaaaa tatgacagaa ctagacactc tt 32
<210> 2
   <211> 32
   <212> DNA
   <213> Artificial Sequence
<220>
   <223> Synthetic Oligonucleotide
<400> 2
   aaaaaaaaaa gacgctaata gttaagacgc ttuuu 32
<210> 3
   <211> 65
   <212> DNA
   <213> Artificial Sequence
<220>
   <223> Synthetic Oligonucleotide
<400> 3
   ctattagcgt ccagtgaatg cgagtccgtc taagagagta gtacagcagc cgtcaagagt 60
   gtcta 65
<210> 4
   <211> 25
   <212> DNA
   <213> Artificial Sequence
<220>
   <223> Synthetic Oligonucleotide
<400> 4
   gttctgtcat atttaagcgt cttaa 25
<210> 5
   <211> 20
   <212> DNA
   <213> Artificial Sequence
<220>
   <223> Synthetic Oligonucleotide
<400> 5
   cagtgaatgc gagtccgtct 20
<210> 6
   <211> 26
   <212> DNA
   <213> Artificial Sequence
<220>
   <223> Synthetic Oligonucleotide
<400> 6
   aaaaaacagt gaatgcgagt ccgtct 26
<210> 7
   <211> 29
   <212> DNA
   <213> Artificial Sequence
<220>
   <223> Synthetic Oligonucleotide
<400> 7
   aaaaaaaaac agtgaatgcg agtccgtct 29